# EUROPÄISCHE PATENTSCHRIFT

(11) **EP 0 807 432 B1**
(45) Veröffentlichungstag und Bekanntmachung des Hinweises auf die Patenterteilung: **17.07.2002**
(21) Anmeldenummer: 97105972.0
(22) Anmeldetag: 11.04.1997
(51) Int. Cl.: A61K 9/22, A61L 27/00, A61K 9/00, A61K 9/20

(54) **Verfahren zur Herstellung eines calciumsulfathaltigen medizinischen Präparats und calciumsulfathaltiges medizinisches Präparat**
Process for the production of a medical preparation containing calcium sulphate and a medical preparation containing calcium sulphate
Procédé pour la fabrication d'une préparation médicale contenant du sulfate de calcium et une préparation médicale contenant du sulfate de calcium

(30) Priorität: 18.05.1996 DE 19620117
(43) Veröffentlichungstag der Anmeldung: 19.11.1997
(73) Patentinhaber: Corimed Kundenorientierte Medizinprodukte GmbH, 64807 Dieburg (DE)
(72) Erfinder: Bauer, Hans Jörg, Dipl.-Ing., 55234 Flomborn (DE); Dingeldein, Elvira, Dr., 63303 Dreieich (DE); Malzer, Wolfgang, 64295 Darmstadt (DE); Wüst, Edgar, 63110 Rodgau (DE); Sattig, Christoph, 64807 Dieburg (DE)
(74) Vertreter: Helber, Friedrich G., Dipl.-Ing.

(56) Entgegenhaltungen:
- EP-A- 0 159 087
- EP-A- 0 159 089
- WO-A-91/17722
- WO-A-96/27346
- GB-A- 2 093 348

## Beschreibung

Die Erfindung betrifft ein Verfahren zur Herstellung eines calciumsulfathaltigen medizinischen Präparats und ein nach dem Verfahren hergestelltes medizinisches Präparat.

Calciumsulfathaltige Präparate und Formkörper besitzen Eigenschaften, die bei den verschiedensten Anwendungen, insbesondere Anwendungen zur äußerlichen oder innerlichen Heilbehandlung des menschlichen oder tierischen Körpers positiv zum Tragen kommen können. So ist es zum Beispiel seit langem bekannt, aus einem Calciumsulfathalbhydrat Gipsverbände zur Ruhigstellung gebrochener Gliedmaßen herzustellen. Da sich Calciumsulfat, insbesondere Calsiumsulfathalbhydrat aufgrund seiner Adsorptionsfähigkeit als Trägermaterial für verschiedene therapeutische Wirkstoffe eignet und zudem vom menschlichen oder tierischen Körper ohne bekannten Nebenwirkungen vollständig resorbiert wird, wurden ferner Versuche unternommen, wirkstoffgetränkte calciumsulfathaltige Formkörper herzustellen und diese dann zwecks Freisetzung der Wirkstoffe zu implantieren. Diese Versuche scheiterten jedoch, da es bislang nicht gelang, chemisch hochreine und sterile Formkörper mit einstellbarer Porösität und Wirkstoff-Rückhaltefähigkeit herzustellen, die den Richtlinien der Medizinprodukteverordnung und des Arzneimittelrechts genügen. Zur Verringerung einer zu schnellen Resorption von Implantaten aus Calciumsulfat wurde auch bereits vorgeschlagen (EP- 059 087 A1), die Implantate aus einem Gemisch von pulverförmigem Calciumsulfat und Calciumcarbonat sowie - gegebenenfalls - unter Zugabe eines medizinischen Wirkstoffs aufzubereiten.

Der Erfindung liegt die Aufgabe zugrunde, ein - erforderlichenfalls auch als implantierbarer Formkörper herstellbares medizinisches Präparat und ein Verfahren zu dessen Herstellung anzugeben, die es erlauben, die genannten positiven Eigenschaften von Calciumsulfat, insbesondere dessen Biokompatibilität für die verschiedensten medizinischen Zwecke nutzbar zu machen und gleichzeitig den an medizinische Präparate gestellten Anforderungen hinsichtlich Reinheit, Reproduzierbarkeit, Verträglichkeit und Standardisierbarkeit zu genügen.

Diese Aufgabe wird erfindungsgemäß gelöst durch ein Verfahren mit den folgenden Schritten:
a) Strahlensterilisation eines pulverförmigen hochreinen Calciumsulfathalbhydrates ;
b) Vermengen von 4 bis 6 Gewichtsteilen des sterilisierten Calciumsulfathalbhydrates mit 5 bis 3 Gewichtsteilen sterilem Wasser in einem Behälter;
c) Schließen des Behälters und Absenken des Drucks in dem Behälter von Umgebungsdruck auf Werte im Bereich zwischen 1 und 500 mbar;
d) Herstellen einer Mischung durch Verrühren des Gemenges für etwa 1 bis 3 Minuten;
e) Erhöhen des Drucks im Behälter auf Umgebungsdruck.

Dabei kann die Saugfähigkeit des auf diese Weise erhältlichen medizinischen Präparats über die zugesetzte Wassermenge sehr genau eingestellt werden, da sich aufgrund des erzeugten Unterdrucks nahezu keine unerwünschten und die Saugfähigkeit verändernden Blasen im Präparat bilden.

Bei der Strahlensterilisation können unterschiedliche Strahlensterilisationsmethoden, also insbesondere Alpha-, Beta- und Gamma-Strahlensterilisation angewandt werden, wobei sich insbesondere die Gamma-Strahlensterilisation bewährt hat. Die Strahlensterilisation hat gegenüber der bekannten Sterilisationsmethode von Calciumsulfathalbhydraten durch Begasung mit Ethylenoxid den Vorteil, daß das Calciumsulfathalbhydrat, das ja eine große Adsorptionsfähigkeit besitzt, nicht mit fremden Stoffen, wie z.B. Ethylenoxid in Berührung kommt, die dann von dem Calciumsulfathalbhydrat adsorbiert werden. Die herkömmliche Methode der Begasung mit Ethylenoxid führt zu so hohen Restmengen an Ethylenoxid im begasten Calciumsulfathalbhydrat, daß dieses nach der Restmengenverordnung und den Medizinprodukte-Richtlinien nicht mehr zur Herstellung von bestimmten medizinischen Präparaten und Formkörpern verwendet werden darf. Die ebenfalls häufig zur Sterilisation verwendete Methode der Autoklavierung kommt bei dem Werkstoff Calciumsulfathalbhydrat nicht in Frage, da dieses unter gespannter Wasserdampfatmosphäre abbindet und sich außerdem bei ca. 140 Grad Celsius in das nicht mehr abbindefähige Calciumsulfathydrat umformt. Die erfindungsgemäß verwendete Sterilisationsmethode ist im industriellen Maßstab auf dieses Produkt anwendbar und birgt keine Gefahr für Produkt oder Patienten. Als Ausgangsmaterial kann ein zur Herstellung von Schlickergießformen in der keramischen Industrie verwendetes hochreines Calciumsulfathalbhydrat verwendet werden.

Bei der Durchführung des Verfahrens wird mit Vorteil so verfahren, daß die Absenkung des Drucks in dem Behälter vorzugsweise auf 50 bis 150 mbar und innerhalb einer Zeitspanne von etwa 30 bis 90 Sekunden, vorzugsweise innerhalb von etwa einer Minute, erfolgt. Dabei kann es zweckmäßig sein, wenn das Gemenge aus Calciumsulfathalbhydrat und Wasser in dem Behälter vor der Absenkung des Drucks für eine Zeitspanne von etwa 30 bis 90 Sekunden, vorzugsweise etwa einer Minute, ruhengelassen wird.

Bevorzugt erfolgt das Verrühren des Gemenges unter Aufrechterhaltung eines Unterdrucks in dem Behälter. Dies hat den Vorteil, daß eventuell noch in dem Gemenge befindliche Luftbläschen spätestens jetzt aus dem Gemenge austreten.

Je nach Anwendungszweck des mit diesem Verfahren herstellbaren calciumsulfathaltigen medizinischen Präparats kann ein therapeutischer Wirkstoff oder eine therapeutische Wirkstoffkombination dem sterilen Wasser und/oder dem Calciumsulfathalbhydrat vor dem Vermengen zugesetzt werden. Wasserunlösliche Stoffe werden dabei vorzugsweise dem pulverförmigen Calciumsulfathalbhydrat zugegeben, während wasserlösliche Stoffe dem sterilen Wasser zugemischt werden. In Wasser gelöste Stoffe werden ebenfalls dem sterilen Wasser zugegeben, wobei dann jedoch eine Reduktion des entsprechenden Teils des Wassers um den in dem gelösten Wirkstoff enthaltenen Wasseranteil erfolgt.

Ein erfindungsgemäßes calciumsulfathaltiges medizinisches Präparat ist z.B. erhältlich durch
a) Strahlensterilisation eines pulverförmigen hochreinen Calciumsulfathalbhydrates;
b) Einstreuen von 50 g des sterilisierten Calciumsulfathalbhydrates in 40 ml in einem Behälter mit einer Rührvorrichtung befindliches steriles Wasser;
c) Ruhelassen der sich durch das Einstreuen ergebenden Mischung aus Calciumsulfathalbhydrat und Wasser für eine Minute;
d) im wesentlichen luftdichtes Verschließen des Behälters und Absenken des Drucks in dem Behälter auf etwa 95 mbar durch etwa einminütiges Absaugen eines wesentlichen Teils der in dem Behälter über der Mischung befindlichen Luft;
e) zweiminütiges Verrühren der Mischung unter Aufrechterhaltung des in dem Behälter erzeugten Unterdrucks;
f) Erhöhen des Drucks in dem Behälter auf Umgebungsdruck und sechsminütiges Ruhenlassen der Mischung.

Durch Beimischen eines therapeutischen Wirkstoffes oder einer therapeutischen Wirkstoffkombination zu dem in dem Behälter befindlichen Wasser vor dem Einstreuen des Calciumsulfathalbhydrates oder zu dem sterilisierten Calciumsulfathalbhydrat ist ein gebrauchsfertiges medizinisches Präparat erhältlich, das hervorragende Eigenschaften bei der Freisetzung des Wirkstoffes bzw. der Wirkstoffkombination besitzt und vom menschlichen Körper vollständig resorbierbar ist.

Das calciumsulfathaltige medizinische Präparat eignet sich sowohl ohne als auch mit einem oder mehreren zugesetzten Wirkstoffen zur Verwendung als therapeutisches Mittel, und zwar insbesondere zur Verwendung als flüssig injizierbares, als in pastösem Zustand einstreichbares oder als in ausgehärtetem Zustand implantierbares therapeutisches Mittel. So ist es z.B. möglich, das Präparat in flüssigem Zustand an einer gewünschten Stelle des menschlichen Körpers zu injizieren, so daß es erst im Körper aushärtet und dann z.B. ein abgesplittertes Knochenteil derart fixiert, daß das Anwachsen des Knochenteiles begünstigt wird. Gegenüber den bislang bekannten in vivo stabilisierenden Teilen wie z.B. Schrauben, Nägel, Platten u.dgl. hat das Präparat den Vorteil, vom Körper vollständig resorbiert zu werden, so daß z.B. nach dem Anwachsen eines abgesplitterten Knochenteiles - anders als bei den genannten Schrauben, Nägeln u.dgl. - kein zweiter Eingriff zur Entfernung der stabilisierenden Teile notwendig ist. Gleichzeitig oder alternativ zu dieser fixierenden Wirkung des Präparates kann das Präparat als Trägermatrix für die unterschiedlichsten therapeutischen Wirkstoffe, z.B. Antibiotika, verwendet werden. Bislang erfolgt eine solche Freisetzung von medizinischen Wirkstoffen im wesentlichen durch Kapselung des Wirkstoffes in eine Kunststoffmatrix bzw. durch eine homogene Untermischung unter die gesamte Substanz, bei der es sich in der Regel um einen reinen Kunststoff oder um mit anorganischen Materialien verfüllte Kunststoffe handelt. Verstärkt finden in letzter Zeit sogenannte Lipide Anwendung, die flüssig oder gallertartig durch Einschmieren in offene Wunden oder durch Injektion implantiert werden, dann hydratisieren und sich so bis zu einem gewissen Grade verfestigen. Die Festigkeit und Körperkonsistenz baut sich im Lauf der Zeit ab, wobei jedoch in der Anfangszeit die äußere Form weitestgehend erhalten bleibt und der Resorptionsvorgang in einer allgemeinen Lockerung der gesamten Struktur erfolgt. Erst danach wird die äußere Geometrie nachhaltig abgebaut. Die Freisetzung von medizinischen Wirkstoffen erfolgt bei diesen Implantaten mit einer hohen Anfangsrate und einem schnellen Abklingen mit längerer Nachlaufzeit. Ferner sind sogenannte Zementplomben bekannt, bei denen ein meist wirkstoffhaltiger Knochenzement, der normalerweise zur Fixierung von Prochesen verwendet wird, angerührt und als teigige Masse in den Implantationsort hineingedrückt wird. Mit diesen Zementplomben kann eine Wirkstofffreisetzung über mehrere Wochen hinweg erzielt werden. Zementplomben sind in der Regel resorptionsbeständig, müssen aber nicht unbedingt entfernt werden. Kunststoffe, Lipide und Zementplomben werden vom Organismus zwar als nicht-körperfeindlich toleriert, agieren jedoch stets als Fremdkörper. Im Falle der Lipide erfolgt eine Auflösung der Trägermatrix, so daß sich das Implantat selbst eliminiert, jedoch sind die Abbauprodukte nicht direkt vom Körper verwertbar. Im Falle der Zementplomben liegt das Implantat als Langzeitimplantat im Organismus, wobei austretende Monomere zumindest anfänglich toxisch wirken. Das erfindungsgemäße Präparat erlaubt demgegenüber eine verbesserte Wirkstofffreisetzung auch über einen langen Zeitraum bei hoher Biokompatibilität und wird zudem vom Körper vollständig resorbiert.

Das erfindungsgemäße calciumsulfathaltige medizinische Präparat kann auch als vor dem Implantieren in den Körper ausgehärteter oder abgebundener Formkörper hergestellt werden, wobei der Formkörper im wesentlichen kugelförmige, zylinder- oder quaderförmige Gestalt aufweisen kann. Dabei kann das medizinische Präparat auch aus einer Vielzahl einzelner, aus einem Draht oder einem Faden, vorzugsweise einem Faden aus vollständig resorbierbarem Material aufgerauhter Formkörper bestehen, wofür die einzelnen Formkörper dann zweckmäßig mit einer Durchgangsöffnung zur Durchführung des Fadens oder Drahts versehen sind.

Solche Formkörper sind an gewünschten Stellen im menschlichen oder tierischen Körper implantierbar und geben in genau vorherberechenbarer Weise den jeweiligen Wirkstoff oder die Wirkstoffkombination frei. Gegenüber den bekannten, auf einem Draht aufgefädelten Formkörpern aus Polymethylmetacrylat haben sie den Vorteil, daß'sie vollständig im Körper resorbiert werden können, während die Polymethylmetacrylat-Formkörper nach der Wirkstofffreisetzung wieder aus dem Körper entfernt werden müssen, was einen zweiten Eingriff bedeutet, der schmerzhaft ist und eine heilende Wunde im Innern wieder aufreißt.

Nachfolgend werden einige Beispiele zur Durchführung des Verfahrens sowie zur Anwendung der nach dem genannten Verfahren hergestellten medizinischen Präparate gegeben.

Beispiel 1: Es liegt eine geschlossene Radiustrümmerfraktur des Handgelenks vor. Nach der Korrektur der Gelenkstelle und der Justierung der Trümmerstücke unter Röntgenkontrolle müßte das Gelenk für mehrere Wochen ruhiggestellt und fixiert werden. Verwendet wird hierzu eine feste Fixierung durch einen angelegten Gipsverband. Im Verlauf des Heilungsprozesses erfolgt eine Muskel- und Gewebemigration, so daß das Gelenk in seiner Gipsfixierung Spielraum erhält und keine feste Plazierung der Knochen-Trümmerstücke mehr gewährleisten kann. Als Folge dieser Gipsverbandslockerung kann es nun passieren, daß das Gelenk aus seiner korrigierten Stellung herausrutscht und in unkontrollierter Fehlstellung zusammenwächst. Das erfindungsgemäß zubereitete Präparat läßt sich nun flüssig injizieren und kann eine solche Dislokation verhindern. Gleichzeitig wird es möglich, durch Zugabe von einem therapeutischen Wirkstoff oder einer therapeutischen Wirkstoffkombination einen Infektionsschutz zu gewährleisten und/oder andere Wirkstoffe an der Defektstelle freizusetzen.

Beispiel 2: Zur Behandlung einer Trümmerfraktur des Oberschenkels (offener Bruch) ist es bislang üblich, den Knochen mittels konventioneller Techniken zu schienen, zu nageln und die Trümmerstücke zu fixieren. In das unmittelbare Umfeld des Bruches und des Operationsgebietes werden üblicherweise zur Infektionsprophylaxe ein Antibiotikaträger, meist in Form von Polymethylmetacrylatkugeln, die auf einer Drahtlitze aufgefädelt sind und ein Antibiotikum freisetzen, implantiert. Das Ende der Drahtlitze ragt dabei aus der Wunde heraus. Später wird die Kette an dem Drahtlitzenende aus dem Wundgebiet herausgezogen. Bei einer anderen bekannten Methode wird in das Wundgebiet ein mit Antibiotika getränktes Kollagenvlies implantiert und eine Drainage zur Sekretabführung gelegt. Mittels erfindungsgemäßer wirkstoffhaltiger Formkörper kann die Funktion der Wirkstofffreisetzung nun wie bei den Polymethylmetacrylatkugeln erfolgen, ohne daß das Ende des Implantates aus der Wunde herausragt (was immer eine Infektionsgefahrenstelle darstellt) und ohne daß das Implantat nach seiner Funktionszeit entfernt werden müßte, da es sich selbst auflöst. Anders als bei der Verwendung von Kollagenvliesen kommt es auch nicht zu einer extremen Sekretbildung.

Beispiel 3: Zur in vivo Fixierung eines Bruches werden 50 g strahlensterilisiertes Calciumsulfathalbhydrat in eine Vorlage von 40 ml sterilen Wassers eingestreut. Nach einer Wartezeit von einer Minute wird der Vorlagenbehälter verschlossen und die in dem Vorlagenbehälter befindliche Luft abgesaugt, so daß der Druck in dem Behälter auf etwa 100 mbar absinkt. Nach weiteren zwei Minuten wird unter Aufrechterhaltung des Unterdrucks über einen Zeitraum von zwei Minuten intensiv mit einem in dem Behälter vorgesehenen Rührer das Gemenge aus Calciumsulfathalbhydrat und Wasser durchmischt. Sodann wird der Druck in dem Behälter wieder auf Umgebungsdruck erhöht und die Mischung für etwa 4 Minuten ruhengelassen. Das Gemisch wird über eine Kanüle innerhalb von weiteren etwa 5 Minuten in das Implantationsgebiet gespritzt und die Implantationsstelle wird für ca. 20 Minuten stillgehalten. Danach erfolgt eine äußere Fixierung des Bruches in herkömmlicher Weise, z.B. mittels Gipsverband.

Beispiel 4: Wie Beispiel 3, jedoch werden in dem Wasser vor der Zugabe des Calciumsulfathalbhydrates etwa 4 g Gentamyzinsulfat gelöst.

Beispiel 5: Wie Beispiel 3, jedoch werden dem Wasser vor dem Einstreuen des Calciumsulfathalbhydrates einige µg Knochenwachstumsfaktor zugesetzt.

Beispiel 6: Anmischung des Präparats wie in den Beispielen 3 bis 5, jedoch wird das Präparat nicht über eine Kanüle injiziert, sondern nach dessen Übergang in die pastöse Phase mittels eines Spachtels in eine Defektstrecke gestrichen.

## Patentansprüche

1. Verfahren zur Herstellung eines calciumsulfathaltigen medizinischen Präparates mit den folgenden Schritten:
a) Strahlensterilisation eines pulverförmigen hochreinen Calciumsulfathalbhydrates;
b) Vermengen von 4 bis 6 Gewichtsteilen des sterilisierten Calciumsulfathalbhydrates mit 5 bis 3 Gewichtsteilen sterilem Wasser in einem Behälter;
c) Schließen des Behälters und Absenken des Drucks in dem Behälter von Umgebungsdruck auf Werte im Bereich zwischen 1 und 500 mbar;
d) Herstellen einer Mischung durch Verrühren des Gemenges für etwa 1 bis 3 Minuten;
e) Erhöhen des Drucks im Behälter auf Umgebungsdruck.

2. Verfahren zur Herstellung eines calciumsulfathaltigen medizinischen Präparates nach Anspruch 1, **dadurch gekennzeichnet, daß** die Absenkung des Drucks in dem Behälter vorzugsweise auf 50 bis 150 mbar und innerhalb einer Zeitspanne von etwa 30 bis 90 Sekunden, vorzugsweise innerhalb von etwa 1 Minute, erfolgt.

3. Verfahren zur Herstellung eines calciumsulfathaltigen medizinischen Präparates nach Anspruch 1 oder 2, **gekennzeichnet durch** den zusätzlichen Schritt des Ruhenlassens des Gemenges aus Calciumsulfathalbhydrat und Wasser in dem Behälter vor der Absenkung des Drucks in dem Behälter für eine Zeitspanne von etwa 30 bis 90 Sekunden, vorzugsweise etwa 1 Minute.

4. Verfahren zur Herstellung eines calciumsulfathaltigen medizinischen Präparates nach einem der Ansprüche 1 bis 3, **dadurch gekennzeichnet, daß** das Verrühren des Gemenges unter Aufrechterhaltung eines Unterdrucks in dem Behälter erfolgt.

5. Verfahren zur Herstellung eines calciumsulfathaltigen medizinischen Präparates nach einem der Ansprüche 1 bis 4, **gekennzeichnet durch** den zusätzlichen Schritt des Beimischens eines therapeutischen Wirkstoffes oder einer therapeutischen Wirkstoffkombination zu dem sterilen Wasser vor dem Vermengen mit dem Calciumsulfathalbhydrat.

6. Verfahren zur Herstellung eines calciumsulfathaltigen medizinischen Präparates nach einem der Ansprüche 1 bis 5, **gekennzeichnet durch** den zusätzlichen Schritt der Beimischung eines therapeutischen Wirkstoffes oder einer therapeutischen Wirkstoffkombination zu dem sterilisierten Calciumsulfathalbhydrat vor dem Vermengen mit dem Wasser.

7. Calciumsulfathaltiges medizinisches Präparat;
**erhältlich durch**
a) Strahlensterilisation eines pulverförmigen hochreinen Calciumsulfathalbhydrates;
b) Einstreuen von 50 g des sterilisierten Calciumsulfathalbhydrates in 40 ml in einem Behälter mit einer Rührvorrichtung befindliches steriles Wasser;
c) Ruhenlassen der sich durch das Einstreuen ergebenden Mischung aus Calciumsulfathalbhydrat und Wasser für eine Minute;
d) im wesentlichen luftdichtes Verschließen des Behälters und Absenken des Drucks in dem Behälter auf etwa 95 mbar durch etwa einminütiges Absaugen eines wesentlichen Teils der in dem Behälter über der Mischung befindlichen Luft;
e) zweiminütiges Verrühren der Mischung unter Aufrechterhaltung des in dem Behälter erzeugten Unterdrucks;
f) Erhöhen des Drucks in dem Behälter auf Umgebungsdruck und sechsminütiges Ruhenlassen der Mischung.

8. Calciumsulfathaltiges medizinisches Präparat nach Anspruch 7, erhältlich durch den zusätzlichen Schritt des Beimischens eines therapeutischen Wirkstoffes oder einer therapeutischen Wirkstoffkombination zu dem in dem Behälter befindlichen Wasser vor dem Einstreuen des Calciumsulfathalbhydrates.

9. Calciumsulfathaltiges medizinisches Präparat nach Anspruch 7 oder 8, erhältlich durch den zusätzlichen Schritt der Beimischung eines therapeutischen Wirkstoffes oder einer therapeutischen Wirkstoffkombination zu dem sterilisierten Calciumsulfathalbhydrat vor dem Einstreuen in das Wasser.

10. Calciumsulfathaltiges medizinisches Präparat nach einem der Ansprüche 1 bis 9, **dadurch gekennzeichnet, daß** es als flüssig injizierbares Präparat, als in pastösem Zustand einstreichbares Präparat oder als in ausgehärtetem Zustand implantierbares Mittel formuliert ist.

11. Calciumsulfathaltiges medizinisches Präparat nach einem der Ansprüche 1 bis 10, **dadurch gekennzeichnet, daß** es im wesentlichen kugelförmige, zylinder- oder quaderförmige Gestalt aufweist.

12. Calciumsulfathaltiges medizinisches Präparat nach einem der Ansprüche 1 bis 11, **dadurch gekennzeichnet, daß** der Formkörper eine Durchgangsöffnung zur Durchführung eines Fadens oder Drahtes aufweist.

## Claims

1. Method of manufacturing a medicinal preparation containing calcium sulphate with the following steps:
a) radiation sterilisation of a pulverulent, highly pure calcium sulphate hemihydrate;
b) mixing 4 to 6 parts by weight of the sterilised calcium sulphate hemihydrate with 5 to 3 parts by weight sterile water in a vessel;
c) closing the vessel and reducing the pressure in the vessel from ambient pressure to values in the range between 1 and 500 mbar;
d) producing a blend by stirring the mixture for about 1 to 3 minutes;
e) increasing the pressure in the vessel to ambient pressure.

2. Method of manufacturing a medicinal preparation containing calcium sulphate as claimed in Claim 1, **characterised in that** the reduction of the pressure in the vessel is effected preferably to 50 to 150 mbar and within a period of time of about 30 to 90 seconds, preferably within about 1 minute.

3. Method of manufacturing a medicinal preparation containing calcium sulphate as claimed in Claim 1 or 2, **characterised by** the additional step of letting the blend of calcium sulphate hemihydrate and water rest in the vessel before reducing the pressure in the vessel for a period of time of about 30 to 90 seconds, preferably about 1 minute.

4. Method of manufacturing a medicinal preparation containing calcium sulphate as claimed in one of Claims 1 to 3, **characterised in that** the stirring of the blend is effected whilst maintaining a reduced pressure in the vessel.

5. Method of manufacturing a medicinal preparation containing calcium sulphate as claimed in one of Claims 1 to 5, **characterised by** the additional step of mixing a therapeutically active material or a combination of therapeutically active materials into the sterile water before mixing with the calcium sulphate hemihydrate.

6. Method of manufacturing a medicinal preparation containing calcium sulphate as claimed in one of Claims 1 to 5, **characterised by** the additional step of mixing a therapeutically active material or a combination of therapeutically active materials into the sterilised calcium sulphate hemihydrate before mixing with the water.

7. Medicinal preparation containing calcium sulphate obtainable by
a) radiation sterilisation of a pulverulent, highly pure calcium sulphate hemihydrate;
b) feeding 50 g of the sterilised calcium sulphate hemihydrate into 40 ml sterile water in a container with a stirrer;
c) letting the blend of calcium sulphate hemihydrate and water produced by the feeding rest for 1 minute;
d) closing the vessel in a substantially air-tight manner and reducing the pressure in the vessel to about 95 mbar by exhausting a substantial proportion of the air within the vessel above the mixture for about 1 minute;
e) stirring the mixture for 2 minutes whilst maintaining the reduced pressure produced in the vessel;
f) increasing the pressure in the vessel to ambient pressure and letting the blend rest for 6 minutes.

8. Medicinal preparation containing calcium sulphate as claimed in Claim 7 obtainable by the additional step of mixing a therapeutically active substance or a combination of therapeutically active substances into the water in the vessel before feeding in the calcium sulphate hemihydrate.

9. Medicinal preparation containing calcium sulphate as claimed in Claim 7 or 8 obtainable by the additional step of mixing a therapeutically active material or a combination of therapeutically active materials into the sterilised calcium sulphate hemihydrate before feeding in the water.

10. Medicinal preparation containing calcium sulphate as claimed in one of Claims 7 to 9, **characterised in that** it is formulated in the form of a liquid injectable preparation, as a coatable preparation in a pasty state or as an implantable compound in the set state.

11. Medicinal preparation containing calcium sulphate as claimed in one of Claims 7 to 10, **characterised in that** it has a substantially spherical, cylindrical or parallelepiped shape.

12. Medicinal preparation containing calcium sulphate as claimed in one of Claims 7 to 11, **characterised in that** the shaped body has a through opening through which a thread or wire may be passed.

## Revendications

1. Procédé de fabrication d'une préparation médicale contenant du sulfate de calcium, comprenant les étapes suivantes :
a) stérilisation par radiations d'un sulfate de calcium hémihydrate pulvérulent de haute pureté,
b) mélange dans un récipient de 4 à 6 parties en poids du sulfate de calcium hémihydrate stérilisé avec 5 à 3 parties en poids d'eau stérile,
c) fermeture du récipient et abaissement de la pression dans celui-ci de la pression ambiante à des valeurs comprises entre 1 et 500 mbar,
d) préparation d'un mélange par agitation du mélange pendant environ 1 à 3 minutes,
e) élévation de la pression dans le récipient à la pression ambiante.

2. Procédé de fabrication d'une préparation médicale contenant du sulfate de calcium selon la revendication 1, **caractérisé par le fait que** la pression dans le récipient est abaissée de préférence à 50 à 150 mbar et en un laps de temps d'environ 30 à 90 secondes, de préférence en environ 1 minute.

3. Procédé de fabrication d'une préparation médicale contenant du sulfate de calcium selon l'une des revendications 1 et 2, **caractérisé par** l'étape supplémentaire consistant à laisser reposer le mélange de sulfate de calcium hémihydrate et d'eau dans le récipient avant l'abaissement de la pression dans celui-ci en un laps de temps d'environ 30 à 90 secondes, de préférence d'environ 1 minute.

4. Procédé de fabrication d'une préparation médicale contenant du sulfate de calcium selon l'une des revendications 1 à 3, **caractérisé par le fait que** l'agitation du mélange est effectuée avec maintien d'une dépression dans le récipient.

5. Procédé de fabrication d'une préparation médicale contenant du sulfate de calcium selon l'une des revendications 1 à 4, **caractérisé par** l'étape supplémentaire consistant à ajouter un agent thérapeutique ou une combinaison d'agents thérapeutique à l'eau stérile avant le mélange avec le sulfate de calcium hémihydrate.

6. Procédé de fabrication d'une préparation médicale contenant du sulfate de calcium selon l'une des revendications 1 à 5, **caractérisé par** l'étape supplémentaire consistant à ajouter un agent thérapeutique ou une combinaison d'agents thérapeutique au sulfate de calcium hémihydrate stérilisé avant le mélange avec l'eau.

7. Préparation médicale contenant du sulfate de calcium, obtenue par
a) stérilisation par radiations d'un sulfate de calcium hémihydrate pulvérulent de haute pureté,
b) dispersion de 50 g du sulfate de calcium hémihydrate stérilisé dans 40 ml d'eau stérile se trouvant dans un récipient pourvu d'un dispositif d'agitation,
c) repos pendant une minute du mélange de sulfate de calcium hémihydrate et d'eau résultant de la dispersion,
d) fermeture pratiquement étanche à l'air du récipient et abaissement de la pression dans le récipient à environ 95 mbar par aspiration pendant environ une minute d'une partie importante de l'air se trouvant dans le récipient au-dessus du mélange,
e) agitation pendant deux minutes du mélange avec maintien de la dépression produite dans le récipient,
f) élévation de la pression dans le récipient à la pression ambiante et repos du mélange pendant six minutes.

8. Préparation médicale contenant du sulfate de calcium selon la revendication 7, obtenue par l'étape supplémentaire consistant à ajouter avant la dispersion du sulfate de calcium hémihydrate un agent thérapeutique ou une combinaison d'agents thérapeutique à l'eau se trouvant dans le récipient.

9. Préparation médicale contenant du sulfate de calcium selon l'une des revendications 7 et 8, obtenue par l'étape supplémentaire consistant à ajouter avant la dispersion dans l'eau un agent thérapeutique ou une combinaison d'agents thérapeutique au sulfate de calcium hémihydrate stérilisé.

10. Préparation médicale contenant du sulfate de calcium selon l'une des revendications 1 à 9, **caractérisée par le fait qu'**elle est formulée sous forme de préparation injectable à l'état liquide, sous forme de préparation applicable à l'état pâteux ou sous forme de produit implantable à l'état durci.

11. Préparation médicale contenant du sulfate de calcium selon l'une des revendications 1 à 10, **caractérisée par le fait qu'**elle présente une forme sensiblement sphérique, cylindrique ou parallélépipédique.

12. Préparation médicale contenant du sulfate de calcium selon l'une des revendications 1 à 11, **caractérisée par le fait que** le corps façonné présente une ouverture traversante pour le passage d'un fil textile ou métallique.
